(19) Europäisches Patentamt
European Patent Office
Office européen des brevets

(11) Publication number : **0 329 460 B1**

# (12) EUROPEAN PATENT SPECIFICATION

(45) Date of publication of patent specification : **20.05.92 Bulletin 92/21**

(51) Int. Cl.$^5$ : **A61K 31/365,** A61K 31/71, A61K 47/06, A01N 43/90

(21) Application number : **89301547.9**

(22) Date of filing : **17.02.89**

(54) **Stabilised macrolide compositions.**

(30) Priority : **18.02.88 GB 8803836**

(43) Date of publication of application : **23.08.89 Bulletin 89/34**

(45) Publication of the grant of the patent : **20.05.92 Bulletin 92/21**

(84) Designated Contracting States : **AT BE CH DE ES FR GB GR IT LI LU NL SE**

(56) References cited :
**EP-A- 0 045 655**
**EP-A- 0 102 721**
**EP-A- 0 116 401**
**EP-A- 0 146 414**
**CH-A- 408 284**
**FR-A- 2 570 390**

(73) Proprietor : **AMERICAN CYANAMID COMPANY**
**One Cyanamid Plaza**
**Wayne New Jersey 07470 (US)**

(72) Inventor : **Eastlick, David Thomas**
**Benrigg Highfield Road**
**Grance-over-Sands Cumbria (GB)**

(74) Representative : **Skailes, Humphrey John et al**
**Frank B. Dehn & Co. Imperial House 15-19 Kingsway**
**London WC2B 6UZ (GB)**

**EP 0 329 460 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to improvements in the stability of antibiotic compounds.

UK Patent Specification Nos 2166436, 2176182 and 2187742 and European Patent Specification No. 170006 describe antibiotic compounds, designated Antibiotics S541, prepared by fermentation of Streptomyces microorganisms and chemical derivatives thereof. Such compounds have antibiotic, and, in particular, anti-endoparasitic, anti-ectoparasitic, anti-fungal, insecticidal, nematicidal and acaricidal activity and are of special interest for use in agriculture, horticulture and animal and human health. They are also of use as intermediates in the preparation of other active compounds.

We have discovered that these antibiotic compounds tend to be unstable under normal conditions of preparation, use and storage. We have now found that the stability of the compounds can be considerably enhanced when they are in the presence of an antioxidant. Thus, any loss due to instability of the compounds during preparation can be minimised by addition of an antioxidant. In a similar way, the shelf-life of the compounds can be increased if admixed with an antioxidant, thereby allowing for the compounds to be prepared well in advance of their intended use. In the presence of an an tioxidant, the compounds also have increased protection against photodegradation, and this allows for the compounds to be readily stored.

Thus, according to one aspect of the invention, we provide a composition containing an antibiotic S541 compound preparable by fermentation of a Streptomyces microorganism or a chemical derivative thereof in the presence of an antioxidant as defined below.

The fermented compounds will in general be Antibiotic S541 compounds or derivatives thereof produced by an Antibiotics S541 producing microorganism belonging to the genus Streptomyces, especially an Antibiotics S541 producing strain of the species Streptomyces thermarchaensis or Streptomyces cyaneogriseus noncyanogenus. Particular examples of suitable strains include Streptomyces thermarchaensis NCIB 12015 [deposited 10th September 1984], Streptomyces thermarchaensis NCIB 12111, NCIB 12112, NCIB 12113, NCIB 12114 [all deposited 26th June 1985] and Streptomyces cyaneogriseus noncyanogenus NRRL 15773 [deposited 3rd May 1984] and mutants of all these strains.

Particular fermented compounds which may be recovered have the formula (I)

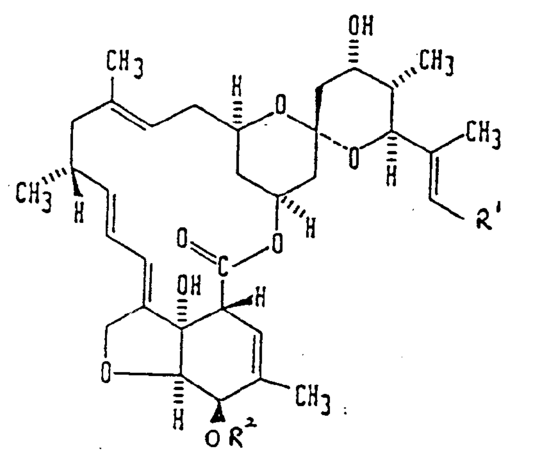

(I)

(where $R^1$ is a methyl, ethyl or isopropyl group and $R^2$ is a hydrogen atom or a methyl group).

An important group of derivatives which may be used in the compositions of the invention is described in GB 2192630A, particularly 23[E]-methoxyimino Factor A.

The antioxidant for use in the composition according to the invention will in general be an antioxidant that is capable of reacting with free radicals, and may be a $C_{1-12}$ alkyl gallate such as ethyl, propyl, octyl or dodecyl gallate; benzyl hydroxybenzoate; butylated hydroxyanisole; butylated hydroxytoluene; quinones and salts thereof, for example $C_{1-6}$ alkyl hydroquinones such as t-butyl hydroquinone and salts thereof, eg the sodium salts; nordihydroguaiaretic acid; or tocopherols such as α-tocopherol. We have found butylated hydroxytoluene to be particularly useful.

The antioxidant may be present in the compositions according to the invention in amounts ranging from 0.005 to 1%, especially 0.02 to 0.3% with respect to the antibiotic compounds. If desired, a mixture of antioxidants may be present in the compositions.

The antibiotic compounds may be in a partially or wholly purified form either as a solid or as a solution in

a suitable solvent, for example a ketone such as acetone, an alcohol such as methanol, a hydrocarbon such as hexane, a halogenated hydrocarbon such as chloroform or methylene chloride, an ester such as ethyl acetate, or acetonitrile. Suitable methods for the preparation of the antibiotic compounds in these forms are described in UK Patent Specification Nos. 2166436, 2176182 and 2187742 and European Patent Specification No. 170006.

Where the compositions of the invention are to be used in human or veterinary medicine, or in agriculture, horticulture or forestry they may also contain one or more suitable carriers or excipients. Thus in a further aspect of the invention we provide a composition comprising an antibiotic compound preparable by fermentation of a Streptomyces microorganism or a chemical derivatives thereof and an antioxidant together with one or more carriers or excipients.

Examples of suitable carriers and excipients are those described in the aforementioned UK and European Patent specifications.

Where the compositions according to the invention have antibiotic activity e.g. antihelminthic activity, for example against nematodes, and in particular, anti-endoparasitic and anti-ectoparasitic activity, they can be used in the treatment of animals and humans with endoparasitic, ectoparasitic and/or fungal infections and in agriculture, horticulture and forestry as pesticides to combat insect, acarine and nematode pests. They may also be used generally as pesticides to combat or control pests in other circumstances, e.g. in stores, buildings or other public places or location of the pests.

Thus according to a further aspect of the invention we provide a composition comprising an antibiotic S541 compound preparable by fermentation of a Streptomyces microorganism or a chemical derivative thereof and an antioxidant optionally also containing one or more carriers or excipients for use as an antibiotic in the treatment of humans or animals or for combatting pests, for example in agriculture, horticulture or forestry.

In general, the compositions may be applied either to the host (animal or human or plants or other vegetation) or to the pests themselves or a locus thereof in accordance with conventional practice.

The compositions according to the invention may be prepared by admixture of the desired ingredients, and according to a further aspect of the invention we provide a process for the preparation of a composition comprising admixing an antibiotic compound preparable by fermentation of a Streptomyces microorganism or a chemical derivative thereof and an antioxidant together, where desired, with one or more carriers or excipients.

The compositions may be prepared by mixing or blending the ingredients in a conventional manner. Thus, in one embodiment a suitable antibiotic compound in a partially purified form in solution may be treated with the antioxidant and, if desired, subsequently co-precipitated from the resulting solution or suspension by the addition or an anti-solvent or by pH adjustment. In another embodiment, a suitable antibiotic compound in a partially or wholly purified form as a solid may be blended with the antioxidant, together, where desired, with one or more carriers or excipients by intimate mixing.

The fermented antibiotic compounds may be isolated from fermentation broth using the methods described in UK Patent Specification 2166436, 2176182 or 2187742 or European Patent Specification 170006. In a further aspect of the present invention we provide for the isolation of an antibiotic S541 compound prepared by fermentation of a Streptomyces microorganisms in the presence of an antioxidant.

According to a further aspect of the invention we provide a method of stabilising Antibiotics S541 compounds preparable by fermentation of a Streptomyces microorganism or chemical derivatives thereof which comprises contacting the said compound with an antioxidant in any conventional way.

The following Examples illustrate the invention. All temperatures are in °C.

In the following Examples 1 to 5 the increased stability of Factor A [a compound of Formula (I) in which $R^1$ is an isopropyl group and $R^2$ is a hydrogen atom] is demonstrated by comparing changes in potency using accelerated temperature techniques. Potency was measured by high performance liquid chromatography using a Spherisorb ODS2 chromatograph.

## Example 1

A sample of Factor A (in a partially purified form) was dissolved in acetone to give a 5% w/v solution. The solution was divided into aliquots. To one aliquot was added butylated hydroxytoluene (25 ppm with respect to the volume of acetone), while nothing was added to a second aliquot. Both aliquots were separately precipitated by the simultaneous addition of the acetone solution (1 volume) and cold water containing 1% v/v sulphuric acid (3 volumes) to a stirred vessel, maintaining the temperature at 0-5°. The solid was filtered, washed with 3 volumes of cold water and dried.

A portion of each solid was heated at 50° for two weeks in a sealed vial, followed by reassay. The following results were obtained.

| Added butylated hydroxytoluene (ppm) | None | 25 |
|---|---|---|
| Change in (%) Potency | -26.3 | -1.1 |

Example 2

Following the method of Example 1 using butylated hydroxytoluene (250 ppm with respect to the volume of acetone) the following results were obtained.

| Added butylated hydroxytoluene (ppm) | None | 250 |
|---|---|---|
| Change in (%) Potency | -36.8 | no change |

Example 3

Following the method of Example 1 using propyl gallate (250 ppm with respect to the volume of acetone) the following results were obtained.

| Added propyl gallate (ppm) | None | 250 |
|---|---|---|
| Change in (%) Potency | -27.3 | -8.8 |

Example 4

Following the method of Example 1 using t-butyl hydroquinone (250 ppm with respect to the volume of acetone) the following results were obtained.

| Added t-butyl hydroquinone (ppm) | None | 250 |
|---|---|---|
| Change in (%) Potency | -27.3 | -8.8 |

Example 5

Dry Factor A was blended with butylated hydroxytoluene (250 ppm) by shaking followed by intimate mixing in a mortar and pestle. A portion of the solid, along with a portion to which no butylated hydroxytoluene had been added, was heated at 50° for two weeks in a sealed vial followed by reassay. The following results were obtained:

| Added butylated hydroxytoluene (ppm) | None | 250 |
|---|---|---|
| Change in Potency (%) | -23.0 | -10.0 |

Example 6

Exposure of Factor A to UV Light

Procedure of Example 5 was followed and the solids either stored under refrigeration or stored at ambient temperature with exposure to ultraviolet light. The following results were obtained:-

| | | MEASURED POTENCY (%) | | | |
|---|---|---|---|---|---|
| | | Start | 3 days | 7 days | 10 days |
| No butylated hydroxy-toluene | Stored under refrigeration | 100.0 | 100.0 | 101.2 | 98.8 |
| | Exposed UV light * Ambient temperature | 100.0 | 92.8 | 83.5 | 84.3 |
| Added butylated hydroxy-toluene (250 ppm) | Stored under refrigeration | 100.0 | 100.0 | 100.5 | 100.1 |
| | Exposed UV light * Ambient temperature | 100.0 | 100.8 | 92.5 | 92.5 |

* A mercury UV lamp (wavelength 366nm)

The following are examples of formulations according to the invention. The term 'Active Ingredient' as used hereinafter means a compound of the formula (I) or a derivative thereof. In all of these compositions an antioxidant is additionally included, e.g. in an amount of 0.02 - 0.3%.

Tablet

Method of manufacture - wet granulation

| | mg |
|---|---|
| Active Ingredient | 250.0 |
| Magnesium stearate | 4.5 |
| Maize starch | 22.5 |
| Sodium starch glycolate | 9.0 |
| Sodium lauryl sulphate | 4.5 |
| Microcrystalline cellulose | to tablet core weight of 450mg |

Add sufficient quantity of a 10% starch paste to the active ingredient to produce a suitable wet mass for granulation. Prepare the granules and dry using a tray or fluid-bed drier. Sift through a sieve, add the remaining ingredients and compress into tablets.

If required, film coat the tablet cores using hydroxypropylmethyl cellulose or other similar film-forming material using either an aqueous or non-aqueous solvent system. A plasticizer and suitable colour may be included in the film-coating solution.

Veterinary tablet for small/domestic animal use

Method of manufacture - dry granulation

| | mg |
|---|---|
| Active Ingredient | 50.0 |
| Magnesium stearate | 7.5 |
| Microcrystalline cellulose to tablet core weight of | 75.0 |

Blend the active ingredient with the magnesium stearate and microcrystallise cellulose. Compact the blend into

slugs. Break down the slugs by passing through a rotary granulator to produce free-flowing granules. Compress into tablets.

The tablet cores can then be film-coated, if desired, as described above.

Veterinary intrammary injection

| | | mg/dose | Range |
|---|---|---|---|
| Active Ingredient | | 150mg | 0.05 - 1.0g |
| Polysorbate 60 | 3.0% w/w) | ) | |
| White Beeswax | 6.0% w/w) | to 3g ) | to 3 or 15g |
| Arachis oil | 91.0% w/w) | ) | |

Heat the arachis oil, white beeswax and polysorbate 60 to 160°C with stirring. Maintain at 160°C for two hours and then cool to room temperature with stirring. Aseptically add the active ingredient to the vehicle and disperse using a high speed mixer. Refine by passing through a colloid mill. Aseptically fill the product into sterile plastic syringes.

Veterinary slow-release bolus

| | % w/w | Range |
|---|---|---|
| Active Ingredient | | 0.25-2g |
| Colloidal silicon dioxide | 2.0) | to required fill weight |
| Microcrystalline cellulose | to 100.0) | |

Blend the active ingredient with the colloidal silicon dioxide and microcrystalline cellulose by using a suitable aliquot blending technique to achieve a satisfactory distribution of active ingredient throughout the carrier. Incorporate into the slow release device and give (1) a constant release of active ingredient or (2) a pulsed release of active ingredient.

Veterinary oral drench

| | % w/v | Range |
|---|---|---|
| Active Ingredient | 0.35 | 0.01 - 2% w/v |
| Polysorbate 85 | 5.0 | |
| Benzyl alcohol | 3.0 | |
| Propylene glycol | 30.0 | |
| Phosphate buffer | as pH 6.0 - 6.5 | |
| Water | to 100.0 | |

Dissolve the active ingredient in the Polysorbate 85, benzyl alcohol and the propylene glycol. Add a proportion of the water and adjust the pH to 6.0 - 6.5 with phosphate buffer, if necessary. Make up to final volume with the water. Fill the product into the drench container.

Veterinary oral paste

| | % w/w | Range |
|---|---|---|
| Active Ingredient | 4.0 | 1 - 20% w/w |
| Saccharin sodium | 2.5 | |
| Polysorbate 85 | 3.0 | |
| Aluminium distearate | 5.0 | |
| Fractionated coconut oil | to 100.0 | |

Disperse the aluminium distearate in the fractionated coconut oil and polysorbate 85 by heating. Cool to room temperature and disperse the saccharin sodium in the oily vehicle. Disperse the active ingredient in the base. Fill into plastic syringes.

Granules for veterinary in-feed administration

| | % w/w | Range |
|---|---|---|
| Active Ingredient | 2.5 | 0.05-5% w/w |
| Calcium sulphate, hemi-hydrate | to 100.0 | |

Blend the Active Ingredient with the calcium sulphate. Prepare the granules using a wet granulated process. Dry using a tray or fluid-bed drier. Fill into the appropriate container.

Veterinary Pour-on

| | % w/v | Range |
|---|---|---|
| Active Ingredient | 2.0 | 0.1 to 30% |
| Dimethyl sulphoxide | 10.0 | |
| Methyl Isobutyl ketone | 30.0 | |
| Propylene glycol (and pigment) | to 100.0 | |

Dissolve the active ingredient in the dimethyl sulphoxide and the methyl isobutyl ketone. Add the pigment and make up to volume with the propylene glycol. Fill into the pour-on container.

Emulsifiable Concentrate

| | |
|---|---|
| Active ingredient | 50g |
| Anionic emulsifier (e.g. Phenyl sulphonate CALX) | 40g |
| Non-ionic emulsifier (e.g. Synperonic NP13) * | 60g |
| Aromatic solvent (e.g. Solvesso 100) to 1 litre. | |

Mix all ingredients, stir until dissolved.

* Trademark of ICI

Granules

(a) Active ingredient 50g
Wood resin 40g
Gypsum granules (20-60 mesh) to 1kg
(e.g. Agsorb 100A)

(b) Active ingredient 50g
Synperonic NP13 * 40g
Gypsum granules (20-60 mesh) to 1kg.

Dissolve all ingredients in a volatile solvent e.g. methylene chloride, add to granules tumbling in mixer. Dry to remove solvent.

* Trademark of ICI

## Claims

1. A stabilised composition comprising a compound of formula (I)

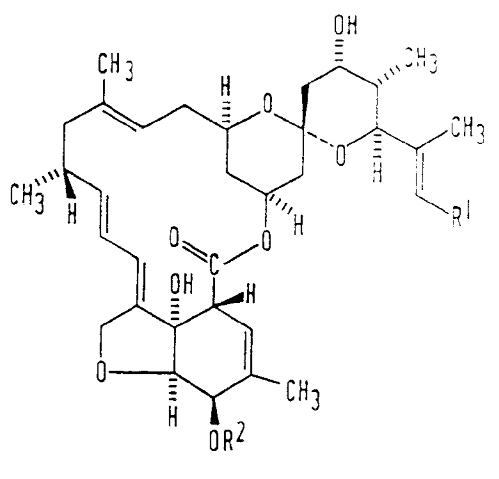

(I)

(where $R^1$ is a methyl, ethyl or isopropyl group and $R^2$ is a hydrogen atom or a methyl group) or a derivative thereof and, as an antioxidant, a $C_{1-12}$ alkyl gallate; benzyl hydroxybenzoate; butylated hydroxyanisole; butylated hydroxytoluene; a quinone or a salt thereof; nordihydroguaiaretic acid; or α-tocopherol.

2. A composition according to claim 1 in which in the compound of formula (I) $R^1$ is an isopropyl group and $R^2$ is a hydrogen atom.

3. A composition according to claim 1 inwhich the antioxidant is butylated hydroxytoluene.

4. A composition according to claim 1 which contains 0.005 to 1% of the antioxidant, by weight of the compound of formula (I) or derivative thereof.

5. A composition according to claim 1 in a form suitable for use as a pesticide and containing one or more carriers or excipients.

6. A method of preparing a composition according to claim 1 which comprises admixing the compound of formula (I) or derivative thereof with the antioxidant.

7. A method according to claim 6 in which said compound and antioxidant are co-precipitated from solution.

8. A method of stabilising a compound of formula (I) as defined in claim 1 or a derivative thereof, which comprises contacting said compound with an antioxidant, said antioxidant being a $C_{1-12}$ alkyl gallate; benzyl hydroxybenzoate; butylated hydroxyanisole; butylated hydroxytoluene; a quinone or a salt thereof; nordihydroguaiaretic acid; or α-tocopherol.

## Patentansprüche

1. Stabilisierte Zusammensetzung, enthaltend eine Verbindung der Formel (I)

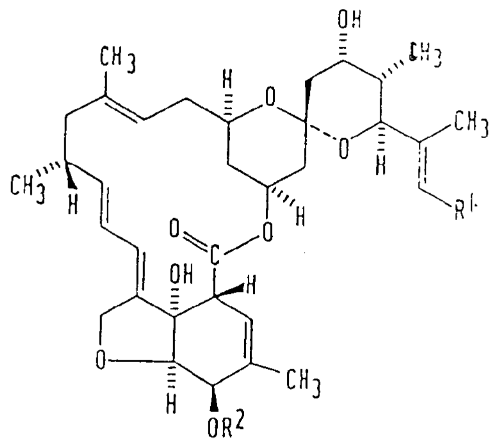

(I)

(worin $R^1$ eine Methyl-, Ethyl- oder Isopropylgruppe bedeutet und $R^2$ für ein Wasserstoffatom oder eine Methylgruppe steht) oder ein Derivat hiervon und als ein Antioxidans ein $C_{1-12}$-Alkyl ga leat;Benzylhydroxybenzoat; butyliertes Hydroxyanisol; butyliertes Hydroxytoluol; ein Chinon oder ein Salz hiervon; Nordihydroguaiaretinsäure; oder α-Tocopherol.

2. Zusammensetzung gemäß Anspruch 1, worin in der Verbindung der Formel (I) $R^1$ für eine Isopropylgruppe steht und $R^2$ ein Wasserstoffatom ist.

3. Zusammensetzung gemäß Anspruch 1, worin das Antioxidans butyliertes Hydroxytoluol ist.

4. Zusammensetzung gemäß Anspruch 1, die 0,005 bis 1 % des Antioxidans, bezogen auf das Gewicht der Verbindung der Formel (I) oder von dessen Derivat, enthält.

5. Zusammensetzung gemäß Anspruch 1 in einer für die Verwendung als Pestizid geeigneten Form, enthaltend einen oder mehrere Träger oder Exzipienten.

6. Verfahren zur Herstellung einer Zusammensetzung gemäß Anspruch 1, das das Mischen einer Verbindung der Formel (I) oder von dessen Derivat mit dem Antioxidans umfaßt.

7. Verfahren gemäß Anspruch 6, worin die Verbindung und das Antioxidans aus einer Lösung copräzipitiert werden.

8. Verfahren zur Stabilisierung einer Verbindung der Formel (I) wie in Anspruch 1 definiert oder eines Derivats hiervon, das das Inkontaktbringen der Verbindung mit einem Antioxidans umfaßt, wobei das Antioxidans ein $C_{1-12}$-Alkylgaleat; Benzylhydroxybenzoat; butyliertes Hydroxyanisol; butyliertes Hydroxytoluol; ein Chinon oder ein Salz hiervon; Nordihydroguaiaretinsäure; oder α-Tocopherol ist.

## Revendications

1. Composition stabilisée comprenant un composé de formule (I)

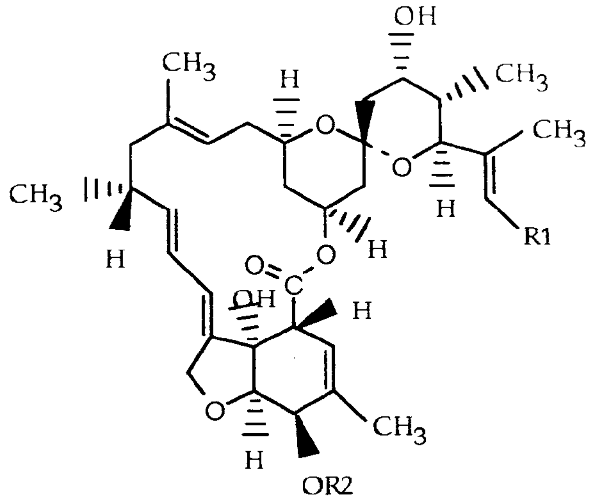

(où $R^1$ est un groupement méthyle, éthyle ou isopropyle et $R^2$ est un atome d'hydrogène ou un groupement méthyle) ou un dérivé de celui-ci et comme antioxydant un gallate d'alkyle en $C_{1-12}$ ; un hydroxybenzoate de benzyle ; un hydroxyanisole butylaté ; un hydroxytoluène butylaté ; une quinone ou un sel de celle-ci ; l'acide nordihydroguaiarétique ; ou l'α-tocophérol.

2. Composition selon la revendication 1 dans laquelle le composé de formule (I) $R^1$ est un groupement isopropyle et $R^2$ un atome d'hydrogène.

3. Composition selon la revendication 1 dans laquelle l'antioxydant est de l'hydroxytoluène butylaté.

4. Composition selon la revendication 1 qui contient de 0,005 à 1% d'antioxydant, par rapport au poids du composé de formule (I) ou d'un dérivé de celui-ci.

5. Composition selon la revendication 1 dans une forme approprié à une utilisation comme pesticide et contenant un ou plusieurs véhicules ou excipients.

6. Méthode de préparation d'une composition selon la revendication 1 qui comprend un mélange du composé de formule (I) ou d'un dérivé de celui-ci avec l'antioxydant.

7. Méthode selon la revendication 6 dans laquelle ledit composé et l'antioxydant sont co-précipités à partir d'une solution.

8. Méthode de stabilisation d'un composé de formule (I) tel que défini dans la revendication 1 ou d'un dérivé de celui-ci qui comprend la mise en contact dudit composé avec un antioxydant, ledit antioxydant étant un gallate d'alkyle en $C_{1-12}$ ; un hydroxybenzoate de benzyle ; un hydroxyanisole butylaté ; un hydroxytoluène butylaté ; une quinone ou un sel de celle-ci ; l'acide nordihydroguaiarétique ; ou l'α-tocophérol.